# FASCICULE DE BREVET EUROPEEN

(11) **EP 2 419 088 B1**
(45) Date de publication et mention de la délivrance du brevet: **05.08.2015**
(21) Numéro de dépôt: 10714898.3
(22) Date de dépôt: 30.03.2010
(51) Int. Cl.: B05D 1/02, A61K 9/16, A61K 9/51

(54) **PROCÉDÉ DE PRÉPARATION DE COMPOSITIONS PHARMACEUTIQUES COMPRENANT DES PARTICULES FINES DE SUBSTANCE ACTIVE**
VERFAHREN ZUR HERSTELLUNG VON PHARMAZEUTISCHEN ZUSAMMENSETZUNGEN MIT FEINEN WIRKSTOFFTEILCHEN
METHOD FOR PREPARING PHARMACEUTICAL COMPOSITIONS COMPRISING FINE PARTICLES OF ACTIVE SUBSTANCE

(30) Priorité: 31.03.2009 FR 0901578
(43) Date de publication de la demande: 22.02.2012
(73) Titulaire: Stanipharm, 54250 Champigneulles (FR)
(72) Inventeur: DESCHAMPS, Frantz, F-54000 Nancy (FR); JUNG, Jennifer, F-54380 Dieulouard (FR); LEBOEUF, Fabrice, F-54130 Saint Max (FR)
(74) Mandataire: Icosa
(86) Numéro de dépôt international: PCT/FR2010/000266
(87) Numéro de publication internationale: WO 2010/112702

(56) Documents cités:
- WO-A-01/43853
- WO-A-2009/034409
- MIZOE ET AL: "Preparation of drug nanoparticle-containing microparticles using a 4-fluid nozzle spray drier for oral, pulmonary, and injection dosage forms", JOURNAL OF CONTROLLED RELEASE, ELSEVIER, AMSTERDAM, NL, vol. 122, no. 1, 22 August 2007 (2007-08-22), pages 10-15, XP022208570, ISSN: 0168-3659, DOI: 10.1016/J.JCONREL.2007.06.001

## Description

La présente invention concerne un procédé de préparation de compositions pharmaceutiques comprenant un ou plusieurs principes actifs sous la forme de particules fines.

Plus particulièrement, la présente invention a pour objet un procédé de production de compositions pharmaceutiques solides comprenant des particules microniques ou submicroniques de principe actif, de préférence des particules cristallines, dispersées à la surface et/ou au sein de poudres d'excipients pharmaceutiques.

### ETAT DE L'ART ANTERIEUR

De nombreuses substances actives, notamment d'intérêt pharmaceutique humain ou vétérinaire, présentent une très faible solubilité dans l'eau et les liquides biologiques aqueux. Cette caractéristique induit une biodisponibilité très faible et souvent fortement variable. La très faible solubilité de ces principes actifs constitue un obstacle majeur à leur évaluation préclinique puis clinique, et bien entendu à leur utilisation à des fins thérapeutiques. De nombreuses substances actives ne peuvent ainsi pas être administrées efficacement à l'aide des formes pharmaceutiques usuelles par voie orale, voie d'administration la plus courante pour laquelle l'absorption du principe actif exige en premier lieu la dissolution de la dose thérapeutique dans les fluides gastro-intestinaux, ou encore par d'autres voies d'administration comme la voie intraveineuse pour laquelle on utilise de manière courante des solutions du principe actif dans des véhicules aqueux.

Des techniques variées ont été étudiées pour produire des formes pharmaceutiques permettant d'administrer des principes actifs peu solubles. On peut citer par exemple les techniques de formulation consistant à produire des solutions micellaires, des dispersions solides dans lesquelles la substance active est dispersée au sein d'une matrice hydrosoluble, des complexes d'association entre la substance active et des cyclodextrines, des formulations lipidiques comme des systèmes auto-émulsionnants, des nanoparticules de lipides solides, des nanocapsules lipidiques et des liposomes. Bien qu'utilisées pour quelques formes pharmaceutiques commerciales, ces techniques de formulation sont souvent complexes, mettent en oeuvre de multiples étapes de fabrication, utilisent le plus souvent des solvants organiques ou des excipients pouvant poser des problèmes de toxicité et peuvent nécessiter le recours à des conditions opératoires sévères (température, cisaillement) pouvant avoir un effet délétère sur la stabilité chimique ou physique du principe actif.

A coté de ces techniques de formulation, une approche souvent efficace consiste à réduire la taille des particules de substance active afin d'augmenter la vitesse de dissolution dans les fluides biologiques. De nombreuses formes pharmaceutiques commerciales contiennent des particules microniques de principe actif, c'est-à-dire des particules de quelques micromètres obtenues par exemple par des procédés de broyage à sec comme le broyage à jet d'air. Comme présenté dans une récente revue de F. Kesisoglou et al. (« Nanosizing - Oral formulation development and biopharmaceutical evaluation », publiée dans Advanced Drug Delivery Reviews, Vol. 59, pp. 631-644, 2007), la production de nanoparticules de substances actives, aussi dénommées particules submicroniques, est un domaine qui connaît un fort et récent développement, notamment parce qu'il a été démontré que la réduction de la taille de particules de principe actif à quelques centaines de nanomètres peut permettre d'augmenter la vitesse de dissolution avec des gains bien plus élevés que ceux obtenus avec des particules micronisées de quelques micromètres en raison de l'effet de la forte augmentation de surface spécifique comme décrit par l'équation de Nernst-Brunner/Noyes-Whitney, mais aussi en raison d'une augmentation théorique de la solubilité à saturation pour des tailles de quelques dizaines ou centaines de nanomètres comme décrit par l'équation de Freundlich et Ostwald.

Les procédés d'obtention de nanoparticules de substances actives utilisés pour produire des formes pharmaceutiques commerciales sont des procédés en voie humide d'homogénéisation haute-pression ou de nanobroyage à l'aide d'un broyeur à billes agitées. On connaît l'utilisation de ces techniques pour la production de deux médicaments, le Triglide™ et le TriCor®, ces deux médicaments administrés par voie orale étant des formes pharmaceutiques contenant des nanoparticules de fénofibrate. Ces procédés mécaniques conduisent à l'obtention d'une dispersion de nanoparticules solides de substances actives dans un liquide. Ce liquide est couramment constitué d'un milieu aqueux contenant un ou plusieurs agents de stabilisation de manière à éviter la formation d'agrégats et une séparation de phase. La sélection des excipients permettant de prévenir l'agglomération des nanoparticules ou de maîtriser la croissance des particules par mûrissement d'Ostwald constitue une tâche empirique lourde et complexe. De plus, ces excipients devant nécessairement être sélectionnés parmi les excipients pharmaceutiques de statut réglementaire adapté. Pour une voie d'administration comme la voie parentérale, la faible quantité d'excipients pharmaceutiques disponibles peut conduire à un développement très difficile de ces formulations.

Bien que le stockage sur de courtes durées de dispersions de particules fines dans un véhicule aqueux puisse être difficile en raison de problèmes de stabilité physique ou microbiologique, les suspensions de nanoparticules produites par les procédés mécaniques susmentionnés sont parfois utilisées en l'état pour des tests précliniques ou même pour les premiers essais cliniques. En revanche, les dispersions aqueuses de nanoparticules de substances actives doivent être converties en formes sèches de manière à obtenir une forme pharmaceutique commerciale d'usage aisé et de stabilité acceptable. Cette conversion peut être effectuée à l'aide de procédés usuels de séchage, comme par exemple le séchage par atomisation ou les techniques de lit d'air fluidisé. Le développement de ces formulations sèches est souvent très complexe car il est nécessaire de mettre au point une forme sèche redispersible, c'est-à-dire permettant après administration orale ou reconstitution dans un milieu aqueux de retrouver une dispersion de nanoparticules de caractéristiques identiques à celles présentes avant séchage, ce qui nécessite souvent le recours à de nombreux excipients de formulation. La production de formes pharmaceutiques commerciales à partir de dispersions de nanoparticules de substance active dans un liquide demande donc un travail de développement lourd et largement empirique et conduit à un procédé global de fabrication du médicament complexe et nécessitant de multiples étapes pouvant avoir un effet délétère sur la stabilité chimique ou physique du principe actif et les performances de la formulation.

En vue de produire des particules fines de substances actives, des procédés de précipitation ou de cristallisation de ces substances préalablement mises en solution sont également connus. En complément de la cristallisation à partir de solutions dans un solvant organique qui peut poser de nombreux problèmes pour des substances actives destinées à l'administration chez l'homme, la précipitation de substances actives à partir d'une solution dans un fluide supercritique est étudiée depuis une vingtaine d'année pour produire des poudres de principes actifs comme présenté dans la revue de J. Jung et al. (« Particle Design using Supercritical Fluids : Litterature and Patent Survey », publiée dans Journal of Supercritical Fluids Vol. 20, pp. 179-219, 2001) ou plus récemment dans la revue de E. Rodier et al. (« La génération de solides divisés par voie supercritique : principes de base, considérations sur l'état d'avancement des recherches », publiée dans Cahiers de Formulation, Vol. 14, pp. 90-108, 2008). Les fluides comprimés, particulièrement le dioxyde de carbone (CO₂) supercritique, connaissent en effet des applications croissantes pour de nombreux procédés.

Pour préciser ce qu'est un fluide supercritique, on rappellera tout d'abord les différents états d'un fluide et ses propriétés dans chacun de ces états. On sait que les corps sont généralement connus sous trois états, à savoir solide, liquide ou gazeux et que l'on passe de l'un à l'autre en faisant varier la température et/ou la pression ; outre l'état solide, il existe l'état liquide et l'état gazeux séparés par la courbe de vaporisation/condensation ; mais, dans le diagramme (Pression, Température) il existe un point au-delà duquel on peut passer de l'état liquide à l'état gaz ou vapeur sans passer par une ébullition ou à l'inverse par une condensation : ce point est appelé le point critique. Un fluide supercritique est caractérisé soit par une pression et une température respectivement supérieures à la pression et à la température critiques dans le cas d'un corps pur, soit par un point représentatif (pression, température) situé au-delà de l'enveloppe des points critiques représentés sur un diagramme (pression, température) dans le cas d'un mélange ; il présente alors, pour de très nombreuses substances, un pouvoir solvant sans commune mesure avec celui observé dans ce même fluide à l'état de gaz comprimé. Il en est de même des liquides dits "subcritiques", c'est-à-dire des liquides qui se trouvent dans un état caractérisé soit par une pression supérieure à la pression critique et par une température inférieure à la température critique dans le cas d'un corps pur, soit par une pression supérieure aux pressions critiques et une température inférieure aux températures critiques des composants dans le cas d'un mélange. Les variations importantes et modulables du pouvoir solvant des fluides supercritiques et la séparation aisée du mélange solvant/soluté par simple décompression sont d'ailleurs utilisées dans de nombreux procédés d'extraction (solide/fluide), de fractionnement (liquide/fluide) et de génération de particules.

Il est à noter que les propriétés physico-chimiques du dioxyde de carbone ainsi que ses paramètres critiques (pression critique : 7,4 MPa et température critique : 31°C) en font le solvant préféré dans de nombreuses applications dans la mesure où il offre la possibilité de travailler à température modérée pour des substances actives thermosensibles, d'autant qu'il ne présente pas de toxicité et est disponible à très bas prix en très grande quantité. D'autres fluides peuvent également être utilisés comme le protoxyde d'azote, les hydrocarbures légers ayant deux à quatre atomes de carbone, les éthers et certains hydrocarbures halogénés comme le tétrafluoroéthane (R134a).

En vue de la génération de particules de substances actives, on connaît le procédé connu sous la désignation de « RESS » (Rapid Expansion of Supercritical Solutions que l'on peut traduire par expansion rapide de solutions supercritiques) décrit dans le brevet US 4,582,731, suivant lequel on détend dans une zone de faible pression une solution de la substance active dans un fluide supercritique. La mise en oeuvre courante du procédé RESS consiste en deux opérations successives. Le fluide supercritique est mis en contact avec le produit à atomiser au sein d'un extracteur et la solution supercritique ainsi générée est réchauffée puis détendue via un dispositif de détente au sein d'un récipient maintenue à une pression nettement inférieure à celle régnant dans l'extracteur. Ce procédé permet l'obtention de fines particules dispersées au sein d'un courant gazeux à faible pression. On connaît également une mise en oeuvre particulière du procédé RESS, décrite dans la demande de brevet WO 01/43853, qui consiste à générer des poudres fines de substances actives par détente d'une solution supercritique à travers une buse puis à capter lesdites poudres fines par percolation du courant gazeux chargé des particules ainsi générées à travers un lit récepteur constitué de granules d'un excipient pharmaceutique. Les produits ainsi générés peuvent être utilisés directement pour confectionner des comprimés ou remplir des gélules.

Une des limitations du procédé RESS réside dans le fait que la solution supercritique doit être portée à une température élevée avant sa détente brutale afin de ne passer à aucun moment dans la zone diphasique liquide/vapeur au cours de la détente. Le passage dans la zone diphasique risque en effet de conduire à la redissolution partielle de la substance active dans la phase liquide. Dans le cas le plus fréquent où la détente est opérée rapidement à travers un capillaire et peut alors être considérée comme isenthalpe, des températures en amont du dispositif de détente allant de 140°C à 200°C sont usuellement mises en oeuvre avec le CO₂, ce qui, malgré le très court temps de résidence de la solution supercritique à cette température, peut conduire à une amorce de dégradation de principes actifs thermosensibles.

Une autre limitation possible du procédé RESS concerne la mise en oeuvre du procédé pour les substances actives présentant une diminution de solubilité dans le fluide supercritique avec l'augmentation de la température à la pression à laquelle la solution est obtenue. En effet, l'homme de l'art sait que la solubilité d'une substance active dans un fluide supercritique peut être représentée par la relation dite de Chrastil. Cette relation met en jeu le produit de deux termes aux effets antagonistes lorsque la température augmente. Par conséquent, pour certaines substances actives, il peut exister une gamme de pression et de température au sein de laquelle la solubilité de la substance active dans le fluide supercritique diminue lorsque la température augmente. Dans ce cas, la mise en oeuvre du procédé RESS pose problème lorsque la température d'extraction est inférieure à la température immédiatement en amont du dispositif de détente dans la mesure où la substance active peut précipiter en amont ou dans le dispositif de détente et provoquer le bouchage dudit dispositif.

Une revue des applications du procédé RESS pour la génération de particules submicroniques (M. Türk, « Manufacture of submicron drug particles with enhanced dissolution behaviour by rapid expansion process », J. of Supercritical Fluids, Vol. 47, pp. 537-545, 2009) montre que ce procédé appliqué à de nombreuses substances actives devrait théoriquement conduire à l'obtention de nanoparticules. La détente brutale à une pression proche de la pression atmosphérique d'une solution dans un fluide supercritique conduit en effet à une sursaturation extrêmement élevée et rapide et donc à la précipitation du produit sous forme de particules submicroniques dispersées dans un courant gazeux lors de la détente. Toutefois, il est souvent observé la production de particules microniques et non de particules submicroniques par le procédé RESS, probablement en raison d'une croissance et/ou d'une agglomération des particules au sein du récipient où elles sont générées. Pour s'affranchir de ce problème, des variantes du procédé RESS ont été étudiées. On connaît ainsi les procédés désignés RESAS ou RESOLV, dont le principe et les applications sont détaillés dans la publication susmentionnée de M. Türk, suivant lesquels la solution supercritique est détendue dans une solution aqueuse contenant des agents de stabilisation tensioactifs qui permettent de limiter la croissance et l'agglomération des particules de substances actives et ainsi de produire des nanoparticules de substances actives. Ces procédés conduisent donc à l'obtention d'une dispersion des nanoparticules dans un milieu aqueux contenant des agents de stabilisation. Les produits obtenus par ce procédé sont donc comparables à ceux obtenus par les procédés de broyage en voie humide ou d'homogénéisation haute-pression et souffrent des mêmes limitations pour la production d'une forme pharmaceutique commerciale.

On connaît également un exemple de procédé utilisant les fluides supercritiques pour lequel la détente de la solution supercritique est opérée dans des conditions de pression et de température qui conduisent à l'obtention d'un mélange de CO₂ gazeux et de CO₂ liquide à la sortie de la buse de détente et non d'un courant de CO₂ gazeux (J. Robertson et al., « Recrystallisation of organic compounds using near critical carbon dioxide », Proceedings of the 4th International Symposium on Supercritical Fluids, May 11-14, Sendai, Japan, 1997). L'homme du métier comprend que cette mise en oeuvre très particulière permet de ne pas chauffer excessivement le fluide supercritique, la température d'extraction et la température immédiatement en amont du dispositif de détente étant identiques, et ainsi de traiter des substances actives thermosensibles. Cette publication enseigne en outre que la taille des particules d'un principe actif augmente avec la pression de détente, probablement en raison d'une sursaturation du fluide diminuant lorsqu'il est détendu à des pressions croissantes et à l'augmentation de la proportion de CO₂ liquide avec la pression qui peut, comme attendu par l'homme de l'art en raison du risque de redissolution de la substance active dans le CO₂ liquide, engendrer une croissance des particules par cristallisation secondaire. On ne connaît pas d'exemples de production de particules submicroniques discrètes à l'aide de ce procédé.

Il existe donc un important besoin de nouvelles méthodes de préparation de nanoparticules de substances actives pouvant être aisément utilisées pour la production de formes pharmaceutiques stables, et de manière avantageuse sans avoir recours à l'usage de nombreux excipients ou à des étapes de production qui conduisent à l'obtention de nanoparticules dispersées dans un liquide et permettant en outre de travailler des substances actives thermosensibles.

### BREVE DESCRIPTION DE L'INVENTION

De manière surprenante et inattendue, les inventeurs ont montré qu'il était possible de produire une composition solide comprenant des particules fines, microniques et/ou de préférence submicroniques d'au moins une substance active dispersées à la surface et/ou au sein d'un solide divisé en mettant en oeuvre un procédé remarquable en ce qu'il comprend une étape consistant à détendre une solution, de ladite substance active dans un fluide à pression supercritique, dans une enceinte à des conditions de pression et de température pour lesquelles une partie du fluide se trouve sous forme liquide lors de la détente et remarquable en ce que ladite enceinte contient un solide divisé.

Le procédé objet de l'invention est particulièrement intéressant en ce qu'il permet de produire des particules fines, microniques et de préférence submicroniques, de substances actives thermosensibles de manière avantageuse car il ne nécessite pas le recours à des températures élevées avant l'étape de détente de la solution supercritique.

Le procédé objet de l'invention est également intéressant en ce qu'il permet de produire des particules fines de substances actives dans des conditions pour lesquelles le chauffage de la solution supercritique avant la détente tel que requis dans la mise en oeuvre du procédé RESS n'est pas possible du fait de la diminution de la solubilité de la substance active dans le fluide à pression supercritique avec la température à la pression choisie.

Le procédé objet de l'invention est particulièrement intéressant en ce qu'il permet d'obtenir un produit solide sec contenant des microparticules ou des nanoparticules de substance active et ne nécessite donc pas en aval d'opérations de conversion en forme sèche pour produire une forme pharmaceutique stable. Le procédé objet de l'invention est donc particulièrement avantageux en ce qu'il permet de substituer des processus complexes de fabrication de formes pharmaceutiques nanoparticulaires, composés par exemple pour les procédés les plus usuels d'étapes multiples de réduction de taille en voie humide conduisant à une dispersion de nanoparticules dans un liquide puis de conversion en forme sèche.

De manière avantageuse, les compositions solides obtenues selon le procédé objet de l'invention peuvent être facilement manipulées et converties en formes finales solides destinées à une administration par voie orale, par exemple, sans que cela soit limitatif, des gélules, des comprimés, des formes orodispersibles, des formes sublinguales ou bioadhésives, des poudres à reconstituer sous la forme de suspension buvable, ladite conversion pouvant s'effectuer par des opérations pharmacotechniques unitaires de routine bien maîtrisées par l'industrie pharmaceutique comme par exemple sans que cela soit limitatif des opérations de mélange, de compression, de granulation ou encore de pelliculage. Les formes orales ainsi produites peuvent être, sans apporter de limitation, des formes à libération immédiate, des formes à libération contrôlée ou encore à libération entérique.

De manière avantageuse, les compositions obtenues par le procédé objet de l'invention peuvent en outre être utilisées pour produire des formes finales destinées à des voies d'administration autres que la voie orale, et de manière non limitative la voie injectable, pulmonaire, nasale, rectale, vaginale ou transdermique. Les compositions solides obtenues par le procédé objet de l'invention peuvent être plus particulièrement utilisées de manière aisée pour produire des formes injectables destinées, de manière non limitative, à une administration par voie intraveineuse, intramusculaire, sous-cutanée, intraoculaire ou encore intra-articulaire, l'administration pouvant avoir lieu par injection rapide et brève ou par perfusion lente. Les compositions solides obtenues par le procédé objet de l'invention peuvent en effet être stockées sous forme sèche de grande stabilité, éventuellement après mélange avec des excipients pharmaceutiques, puis dispersées extemporanément dans un véhicule liquide, préférentiellement dans un milieu aqueux qui peut contenir des agents de dispersion et de stabilisation, de manière à produire une dispersion injectable de particules fines, préférentiellement de particules submicroniques.

De manière avantageuse, la mise en oeuvre du procédé objet de l'invention permet de limiter fortement le nombre d'excipients utilisé pour produire une forme pharmaceutique commerciale stable.

De manière avantageuse, le procédé objet de l'invention permet de produire des particules fines, et préférentiellement des particules submicroniques, avec un état solide (cristallinité et polymorphisme) permettant d'obtenir une forme pharmaceutique stable, et de préférence des particules submicroniques cristallines, encore plus préférentiellement des particules cristallines contenant essentiellement la forme cristalline la plus stable.

Par essentiellement, on entend signifier que plus de 80% et préférentiellement plus de 95% des particules cristallines sont sous la forme cristalline la plus stable à la température de stockage préconisée de la forme pharmaceutique commerciale.

### DESCRIPTION DES FIGURES

Figure 1 : Schéma du dispositif utilisé pour la mise en oeuvre du procédé de l'invention
Figure 2 : Profil de distribution granulométrique en volume des particules de substance active produites lors de l'essai 1-1 (Exemple 1)
Figure 3 : Profil de distribution granulométrique en volume des particules de substance active produites lors de l'essai 1-2 (Exemple 1)
Figure 4 : Courbes de dissolution *in vitro* (Exemple 1)
Figure 5 : Comparaison pour 3 prélèvements des profils de distribution granulométrique en volume des particules de nifédipine produites lors de l'essai 2-1 (Exemple 2)
Figure 6 : Comparaison pour 3 prélèvements des profils de distribution granulométrique en volume des particules de nifédipine produites lors de l'essai 2-2 (Exemple 2)
Figure 7 : Profil de distribution granulométrique en volume des particules de sirolimus produites lors de l'essai 3-1 (Exemple 3).
Figure 8 : Profil de distribution granulométrique en volume des particules de sirolimus produites lors de l'essai 3-2 (Exemple 3).
Figure 9 : Figure 4 : Courbes de dissolution *in vitro* (Exemple 3)

### DESCRIPTION DETAILLEE DE L'INVENTION

Cette invention repose sur le fait qu'il est surprenant de constater que la détente d'une solution comprenant au moins une substance active solubilisée dans un fluide à pression supercritique selon des conditions opératoires conduisant lors de la détente à la présence du fluide en partie sous forme liquide permet l'obtention de particules fines de substances actives, et de manière avantageuse de particules submicroniques, lorsque le fluide est détendu dans une enceinte de détente comprenant un solide divisé.

Ainsi, la présente invention concerne notamment un procédé de préparation d'une composition solide comprenant des particules fines, microniques et de préférence submicroniques d'au moins une substance active dispersées à la surface et/ou au sein d'un solide divisé remarquable en ce qu'il comprend une étape consistant à détendre une solution, de ladite substance active dans un fluide à pression supercritique, dans une enceinte à des conditions de pression et de température pour lesquelles une partie du fluide se trouve sous forme liquide lors de la détente et en ce que ladite enceinte contient un solide divisé.

Selon un mode de réalisation préféré, le procédé selon l'invention comprend les étapes consistant à :
a) Former une solution de la substance active dans un fluide à pression supercritique ;
b) Détendre ladite solution dans une enceinte dans des conditions de pression et de température pour lesquelles une partie du fluide se trouve sous forme liquide lors de la détente ;
c) Mettre en contact le fluide ainsi détendu avec un solide divisé dans ladite enceinte ;
d) Récupérer un produit sec contenant ladite substance active sous forme de particules fines et ledit solide divisé.

Selon un mode de réalisation encore plus préféré, le procédé selon l'invention consiste à :
a) Former une solution de la substance active dans un fluide à pression supercritique ;
b) Détendre ladite solution dans une enceinte dans des conditions de pression et de température pour lesquelles une partie du fluide se trouve sous forme liquide lors de la détente ;
c) Mettre en contact le fluide ainsi détendu avec un solide divisé dans ladite enceinte ;
d) Récupérer un produit sec contenant ladite substance active sous forme de particules fines et ledit solide divisé.

Dans le cadre de la présente invention, le terme « partie du fluide se trouve sous forme liquide » signifie qu'au moins 1%, et par ordre de préférence croissante au moins 5%, au moins 10%, au moins 20%, au moins 30%, et au moins 50% du fluide se trouve sous forme liquide lors de la détente. Selon un mode de réalisation préféré, entre 10 et 90% et de façon tout à fait préférée entre 20 et 80% du fluide se trouve sous forme liquide lors de la détente.

Dans le cadre de la présente invention, le pourcentage de fluide sous forme liquide est le pourcentage théorique de fluide sous forme liquide aux conditions de température et de pression de la détente. Celui-ci peut être déterminé sur la base d'hypothèses relatives au mécanisme de détente, par exemple en utilisant un diagramme thermodynamique pression-enthalpie dit « diagramme de Mollier » qui permet de déterminer pour chaque conditions de température et de pression du fluide immédiatement en amont de la détente et de pression dans l'enceinte de détente, le pourcentage de fluide sous forme gazeuse et le pourcentage de fluide sous la forme liquide. De tels diagrammes peuvent être établis par exemple à partir de données thermodynamiques facilement accessibles à l'homme de l'art, telles que, E.W. Lemmon, M.O. McLinden and D.G. Friend, "Thermophysical Properties of Fluid Systems" dans le WebBook de Chimie NIST, Base de Données Standard de Référence NIST Numéro 69, Eds. P.J. Linstrom and W.G. Mallard, National Institute of Standards and Technology, Gaithersburg MD, 20899, (http://webbook.nist.gov).

Par souci de clarté, il est précisé que l'autre partie du fluide qui n'est pas sous forme liquide lors de la détente est sous forme gazeuse.

Dans le cadre de la présente invention, le pourcentage de fluide sous forme liquide correspond à des rapports de masses entre la masse de fluide sous la forme liquide et la masse de fluide total.

Dans la suite, on appellera nanoparticules ou particules submicroniques des particules de taille médiane inférieure à 1000 nm. On définit ici des particules de taille médiane inférieure à 1000 nm comme un ensemble de particules dont 50% ont un diamètre volumique inférieur à 1000 nm, ledit diamètre volumique médian étant mesuré par exemple par granulométrie laser ou spectroscopie à corrélation de photons.

Les particules comprenant une substance active obtenues par le procédé objet de l'invention sont de manière avantageuse des particules microniques, de préférence des particules submicroniques, de manière encore plus préférée des particules submicroniques de diamètre volumique médian (dv(0,5)) inférieur à 800 nm, de manière encore plus préférée de diamètre volumique médian inférieur à 500 nm. 90% des particules fines présentent de préférence un diamètre volumique (dv(0,9)) inférieur à 10 µm, de manière encore préférée inférieur à 5 µm, de manière encore plus préférée inférieur à 2 µm, de manière encore plus préférée inférieur à 1000 nm.

Dans le cadre de la présente invention, on appellera fluide à pression supercritique un fluide porté à une pression supérieure à sa pression critique, c'est-à-dire soit un fluide supercritique proprement dit, soit un liquide dit sub-critique comme défini ci-dessus ; on appellera solution supercritique une solution d'une ou plusieurs substance(s) active(s) dans un fluide supercritique et solution à pression supercritique une solution d'une ou plusieurs substance(s) active(s) dans un fluide à pression supercritique.

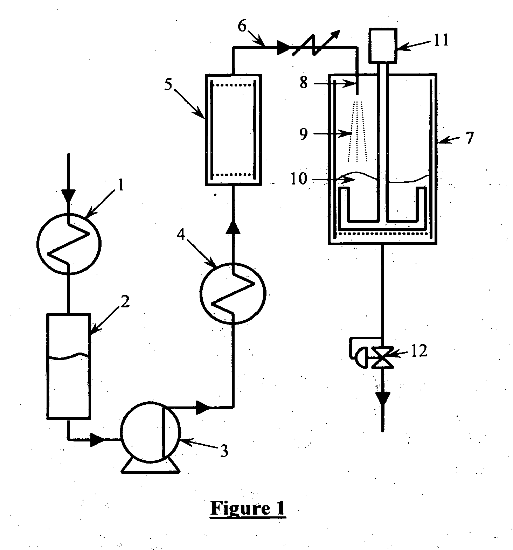

Selon un mode de réalisation de l'invention, on forme une solution dans un fluide à pression supercritique d'au moins une substance active. Cette solution à pression supercritique est produite préférentiellement en utilisant un équipement similaire à celui présenté en Figure 1 pour lequel on introduit la substance active solide dans un extracteur à haute pression (5) au sein duquel on fait percoler le fluide à pression supercritique au sein de l'extracteur. Ce mode de mise en oeuvre correspond à celui couramment utilisé par l'homme de l'art dans les procédés d'extraction par fluide supercritique à partir de matrices solides, la substance active constituant le composé à extraire et la matrice solide étant constituée soit de la substance active solide seule, soit de la substance active mélangée à un matériau solide inerte. L'homme de l'art comprendra que ledit matériau inerte, mélangé de manière uniforme selon les moyens appropriés avec la substance active solide dans les conditions ambiantes, permet principalement d'améliorer l'efficacité du contact entre le fluide à pression supercritique et la substance active, de manière à garantir la mise en solution de la substance active dans le fluide à pression supercritique de manière reproductible. En outre, le matériau inerte peut permettre d'immobiliser la substance active et d'assurer un contact efficace avec le fluide à pression supercritique dans le cas particulier où la substance active, qui est solide dans les conditions ambiantes, se trouve sous forme liquide ou semi-solide dans les conditions d'extraction. A titre d'exemple et de manière non limitative, le matériau inerte peut être constitué de matériaux fibreux, de matériaux poreux ou de billes, de préférence des billes en verre, en céramique, en acier inoxydable, en oxyde de zirconium ou en polymères de diamètre approprié pour assurer un mélange uniforme et reproductible avec la substance active avant mise en oeuvre dans l'extracteur haute pression. Préférentiellement, un ou plusieurs éléments garantissant l'absence d'entraînement de particules de substance active non solubilisées à la sortie de l'extracteur seront utilisés.

Dans un mode de mise en oeuvre préféré du procédé objet de l'invention, l'étape de formation de la solution à pression supercritique permet de garantir une concentration constante en substance active dans le fluide à pression supercritique au cours du procédé. Dans cette perspective et en s'inspirant de ce qui est couramment mis en oeuvre dans les procédés d'extraction à partir de matrices solides, il est possible de mettre en oeuvre la technique que l'homme de l'art connaît sous le nom de lit mobile simulé, consistant à utiliser au minimum trois extracteurs, avec au moins deux extracteurs haute pression en série à tout moment de la mise en oeuvre du procédé et un extracteur en phase de décompression, déchargement, remplissage ou mise sous pression pendant ce temps. Avantageusement, le premier extracteur parmi les extracteurs placés en série est celui le plus proche de l'épuisement en substance active. La mise en oeuvre d'un tel dispositif permet de garantir une concentration constante en substance active dans le fluide à pression supercritique. En outre, sous réserve du dimensionnement de l'installation selon les règles de l'art, la concentration en substance active dans le fluide supercritique peut en effet être alors voisine de la concentration dite à saturation, correspondant à la limite de solubilité de la substance active dans le fluide à pression supercritique dans les conditions d'extraction choisies.

Les mises en oeuvre décrites ci-dessus ont le point commun de conduire généralement à une solution proche de la saturation en substance active dans le fluide à pression supercritique en sortie d'extracteur.

Une mise en oeuvre particulière du procédé objet de l'invention consiste à mélanger avant l'étape de détente la solution dans le fluide à pression supercritique avec une quantité connue de fluide à pression supercritique ne contenant pas de substance active, par exemple par l'intermédiaire d'un mélangeur statique haute pression, afin d'ajuster la concentration en substance active dans le fluide à pression supercritique avant détente et mise en contact avec un solide divisé, de manière à ajuster les propriétés des compositions pharmaceutiques obtenues par le procédé objet de l'invention.

Selon un mode de mise en oeuvre particulier, les dispositifs ci-dessus peuvent être complétés par un système de mesure en ligne de la concentration en substance active dans le fluide à pression supercritique, par exemple sans que cela soit limitatif par une méthode spectrophotométrique.

Selon un mode de réalisation préféré de l'invention, le fluide à pression supercritique est sélectionné parmi le dioxyde de carbone, le protoxyde d'azote, les alcanes comme par exemple l'éthane ou le propane, les éthers comme par exemple le diméthyl éther, les hydrocarbures fluorés comme par exemple le tétrafluoroéthane (R134a) ou le trifluorométhane (R23).

Selon un mode de réalisation tout à fait préféré, le fluide à pression supercritique est le dioxyde de carbone.

Selon un autre mode de réalisation de l'invention, le fluide à pression supercritique est constitué soit d'un mélange d'au moins deux des fluides susmentionnés soit d'un mélange d'un des fluides susmentionnés et d'au moins un solvant organique, choisi avantageusement, sans limitation, parmi les alcools, les cétones, les esters.

Selon un mode de réalisation du procédé selon l'invention, le fluide à pression supercritique est du dioxyde de carbone à une pression comprise entre 7,4 MPa et 200 MPa, préférentiellement entre 10 et 100 MPa et à une température inférieure à 140°C, et par ordre de préférence croissante à une température inférieure à 80°C, 60°C et 40°C.

Selon un mode réalisation préféré selon l'invention, le fluide à pression supercritique est du dioxyde de carbone à une pression comprise entre 25 MPa et 70 MPa, à une température comprise entre 40 et 80°C.

Selon une mise en oeuvre préférée du procédé objet de l'invention, la solution à pression supercritique est composée d'une substance active dissoute dans le fluide à pression supercritique.

Selon une mise en oeuvre particulière du procédé objet de l'invention, la solution à pression supercritique comprend plusieurs solutés, et préférentiellement au moins deux substances actives ou au moins une substance active et un excipient pharmaceutique.

Selon le procédé objet de l'invention, la solution de substance active dans le fluide à pression supercritique est détendue dans une enceinte dans des conditions conduisant lors de la détente à la formation d'un système diphasique comprenant le fluide à l'état liquide et le fluide à l'état gazeux.

La température de la solution à pression supercritique immédiatement en amont du dispositif de détente peut être régulée par exemple et de manière non limitative par le passage de la solution supercritique au sein d'un échangeur. Dans une mise en oeuvre particulière du procédé de l'invention, l'ajustement de la température de la solution à pression supercritique peut être opéré par mélange de la solution à pression supercritique issue de l'extracteur haute pression avec du fluide à pression supercritique à une température différente de la température de la solution à pression supercritique. Dans des conditions que l'homme de l'art identifiera comme correspondant à un régime permanent de fonctionnement du procédé de l'invention, il est donc possible d'ajuster de manière simultanée la concentration en substance active sensiblement en dessous de la concentration dite à saturation dans le fluide à pression supercritique et la température de la solution à pression supercritique immédiatement en amont du dispositif de détente.

Selon une mise en oeuvre particulière de l'invention, la température du fluide à pression supercritique lors de l'étape de formation de la solution à pression supercritique est identique à la température immédiatement en amont du dispositif de détente.

Selon une autre mise en oeuvre de l'invention, la température du fluide à pression supercritique lors de l'étape de formation de la solution à pression supercritique est sensiblement inférieure à la température immédiatement en amont du dispositif de détente.

Selon un mode de réalisation du procédé selon l'invention, la solution à pression supercritique est détendue dans une enceinte à l'aide d'un dispositif de détente constitué d'une buse de pulvérisation, d'un tube, d'un orifice percé dans une plaque de faible épaisseur, d'un élément en matériau solide fritté, d'une vanne à ouverture contrôlée ou par tout autre dispositif connu de l'homme de l'art.

Selon un mode de mise en oeuvre préféré, le dispositif de détente est constitué d'un tube capillaire caractérisé par un rapport de longueur sur diamètre interne supérieur à 20 et de préférence supérieur à 100.

Quel que soit le dispositif de détente utilisé, l'homme de l'art peut aisément déterminer les conditions opératoires du procédé conduisant à la formation d'une partie de fluide à l'état liquide lors de la détente, par exemple par l'examen de diagrammes thermodynamiques tels que ceux dénommés « diagrammes de Mollier » sur la base d'hypothèses relatives au mécanisme de détente, ladite détente pouvant être considérée comme majoritairement isenthalpe ou isentrope selon le dispositif de détente choisi, ou encore au cours de tests préliminaires sur l'appareil permettant la mise en oeuvre du procédé, par exemple et de manière non limitative en mesurant la température immédiatement en aval du dispositif de détente dans l'enceinte de détente ou en mesurant une grandeur physique immédiatement en aval de ce dispositif qui permet de conclure à la présence d'une partie de fluide sous forme liquide.

Selon le procédé objet de l'invention, la pression dans l'enceinte de détente est maintenue à une valeur conduisant à l'obtention d'un milieu diphasique gaz-liquide lors de la détente, en fonction des conditions de pression et de température de la solution à pression supercritique immédiatement en amont du dispositif de détente. Cette pression peut être déterminée par l'homme de l'art par exemple à l'aide de diagrammes thermodynamiques comme les diagrammes pression-enthalpie. La pression peut être maintenue à cette valeur à l'aide d'une vanne régulatrice de pression amont située en aval de l'enceinte de détente ou de tout dispositif connu de l'homme de l'art comme offrant un contrôle de la pression dans ces conditions opératoires.

Le procédé objet de l'invention est préférentiellement conduit avec du dioxyde de carbone avec une pression dans l'enceinte de détente maintenue entre 0,52 et 7,4 MPa et préférentiellement entre 1 et 6,5 MPa.

Selon une mise en oeuvre préférée de l'invention, une partie du fluide qui se trouve à l'état liquide lors de la détente est vaporisée dans l'enceinte de détente en apportant de l'enthalpie par tout moyen connu de l'homme de l'art, de préférence par chauffage des parois de l'enceinte ou par introduction d'un fluide à un débit et une température permettant d'apporter l'enthalpie requise.

Selon un mode de mise en oeuvre préféré, le fluide introduit pour l'apport d'enthalpie est identique au fluide utilisé pour la formation de la solution à pression supercritique.

Selon un mode de mise en oeuvre préféré de l'invention, le fluide à l'état liquide est entièrement vaporisé à l'intérieur de l'enceinte de détente et se trouve par conséquent intégralement à l'état gazeux lorsqu'il quitte ladite enceinte.

Selon le procédé objet de l'invention, le fluide à pression supercritique dans lequel est solubilisée au moins une substance active est détendu dans une enceinte comprenant un solide divisé. Ainsi, ledit solide divisé entre en contact en tout ou partie avec le fluide détendu en partie à l'état liquide. Selon un mode de réalisation préféré de l'invention, ledit solide divisé comprend un excipient pharmaceutique.

Selon un mode de réalisation préféré de l'invention, le fluide détendu est mis en contact avec le solide divisé avec tout moyen connu de l'homme de l'art permettant d'uniformiser la répartition du mélange biphasique gaz-liquide du fluide sur le solide divisé et ainsi de conduire à une répartition uniforme de la substance active dans la composition solide et sèche finale.

Selon un mode de mise en oeuvre encore plus préféré, le positionnement du dispositif de détente et la configuration géométrique de l'enceinte de détente permettent d'obtenir l'agitation du solide divisé par la seule vitesse d'éjection du fluide détendu.

De manière avantageuse, l'enceinte de détente est équipée d'un système d'agitation mécanique permettant si nécessaire d'agiter le solide divisé.

Selon un autre mode de mise en oeuvre du procédé de l'invention, l'enceinte de détente est équipée d'un dispositif permettant sans dépressurisation complète de ladite enceinte l'alimentation en solide divisé et le soutirage de la composition solide comprenant le solide divisé et la substance active. Cette alimentation et ce soutirage sont opérés avantageusement à intervalles de temps réguliers et de préférence en continu. Un tel dispositif permet d'utiliser une enceinte de détente d'un volume sensiblement réduit par rapport à la mise en oeuvre discontinue du procédé.

Selon une mise en oeuvre particulière de l'invention, le solide divisé est une poudre ou un matériau granulaire constitué d'au moins un excipient pharmaceutique.

Selon une mise en oeuvre particulièrement avantageuse de l'invention, le solide divisé est constitué de particules d'au moins un excipient acceptable pour une administration par voie orale, et préférentiellement choisi parmi les excipients connus de l'homme de l'art pour présenter des propriétés favorables pour la production de formes orales solides comme les comprimés ou les gélules. Les excipients pour administration orale sont avantageusement choisis parmi les sucres comme le lactose ou le sucrose, les polysaccharides comme la cellulose microcristalline, les dérivés de cellulose ou l'amidon, les polyols comme le mannitol, les lipides solides et les cires, les homopolymères et les copolymères solides comme les polyesters, les polyéthylène glycols, les poloxamères, les polyvinylpyrrolidones et dérivés, les composés inorganiques comme la silice.

Selon une autre mise en oeuvre avantageuse du procédé objet de l'invention, le solide divisé est constitué d'une poudre composée d'excipient soluble dans les milieux aqueux et acceptable pour une administration par voie injectable comme, sans que cela soit limitatif, les voies intraveineuse, intramusculaire, intra-articulaire ou intraoculaire. Les excipients pour administration par voie injectable sont avantageusement choisis parmi les sels comme le chlorure de sodium, les sucres comme par exemple le tréhalose ou le sucrose, les polyols comme par exemple le mannitol, les polysaccharides, les polymères biorésorbables, les protéines comme l'albumine.

Selon une mise en oeuvre avantageuse du procédé de l'invention, le solide divisé est constitué de particules d'excipient pharmaceutique d'une taille moyenne comprise entre 50 et 2 000 micromètres, et préférentiellement entre 200 et 600 micromètres.

Selon une mise en oeuvre avantageuse du procédé objet de l'invention, le rapport massique entre la substance active et le solide divisé est compris entre 0,1 et 25 %, et préférentiellement entre 0,5 et 10%.

Selon une mise en oeuvre préférée du procédé, on utilise un dispositif tel que décrit sur la Figure 1. La substance active, éventuellement mélangée à un matériau inerte, est placée dans une cellule d'extraction (tube fermé aux extrémités par des frittés en acier inoxydable pour éviter l'entraînement du produit par le fluide). Cette cellule est placée à l'intérieur d'un autoclave d'extraction (5) chauffé à la température d'extraction choisie. Le fluide est pompé (3) depuis le stockage (2) et traverse un échangeur chaud (4) régulé à la température d'extraction désirée avant d'entrer dans l'autoclave d'extraction. La solution de produit solubilisé dans le fluide à pression supercritique sortant de l'autoclave d'extraction est envoyée vers une enceinte de détente (7) dans laquelle la solution est dépressurisée brutalement à une température et une pression donnée à travers une buse (8), ladite buse étant constitué d'un tube long ou d'un orifice percé au laser dans une plaque de faible épaisseur, et de préférence un tube capillaire constitué par un tube de rapport longueur sur diamètre supérieur à 100. Un dispositif de chauffage (6) permet de régler la température du fluide immédiatement en amont du dispositif de détente. Selon les conditions opératoires choisies selon le procédé de l'invention, une partie du fluide se trouve sous forme liquide lors de la détente. Par ailleurs, la pression dans l'enceinte de détente est maintenue à une valeur déterminée grâce à une vanne de régulation de pression amont (12). Cette vanne de régulation permet de régler les conditions opératoires dans l'enceinte de détente indépendamment de la température immédiatement en amont du dispositif de détente réglée par le dispositif de chauffage (6).

L'enceinte de détente contient un panier de collecte, fermé par un filtre, au sein duquel est placée une poudre d'excipient pharmaceutique (10) avant le démarrage du procédé. Selon une mise en oeuvre particulière du procédé, un dispositif d'agitation (11) permet d'homogénéiser le contenu de l'enceinte de détente lors de la mise en oeuvre du procédé.

Par ailleurs, selon la mise en oeuvre préférée du procédé, l'enceinte de détente est chauffée à une température choisie de telle sorte que dans les conditions opératoires mises en oeuvre, au moins une partie du fluide à l'état liquide puisse être vaporisée avant de quitter l'enceinte de détente. Après le passage dans la vanne de régulation (12), le fluide est ensuite évacué dans l'atmosphère ou éventuellement recomprimé et recyclé selon l'art connu.

### Exemples

Les exemples de mise en oeuvre suivant sont présentés afin d'illustrer de façon non limitative le procédé selon l'invention.

### Exemple 1 : Production d'une formulation d'une substance active et de lactose

Le procédé de l'invention est mis en oeuvre sur un équipement correspondant au schéma décrit sur la Figure 1 pour la production d'une formulation constituée de particules submicroniques d'une substance active et de lactose avec un taux de charge massique en substance active visé de 5%.

10 g de substance active mélangé à environ 800 g de billes de verre de 1 mm de diamètre est disposé dans un panier d'extraction de 1,5 L. Ledit panier est placé à l'intérieur de l'autoclave d'extraction (5) chauffé à une température de 50°C. Après une étape de mise en régime, du dioxyde de carbone à l'état supercritique, porté à une pression de 28 MPa via la pompe (3) à un débit de 5 kg/h et à une température de 50°C via l'échangeur (4), percole à travers le panier d'extraction pour extraire la substance active. La solution de substance active dans le dioxyde de carbone supercritique ainsi formée est détendue à travers une buse de pulvérisation (8) constituée d'un capillaire en polyétheréthercétone (PEEK) de diamètre interne 170 µm et de longueur adaptée à la pression et au débit de travail. L'enceinte de détente (7), d'un volume intérieur utile de 545 mL, est chauffée à 50°C. La température de la solution supercritique immédiatement en amont de la buse est réglée à 50°C via le dispositif de chauffage (6). La pression au sein de l'enceinte de détente est réglée à 40 bar par une vanne automatique de régulation de la pression amont (11). Compte tenu des conditions de température et de pression immédiatement en amont de la buse de pulvérisation et de la pression réglée dans l'enceinte de détente, et sachant que le type de buse utilisé permet de considérer la détente comme quasi isenthalpe, il est possible de déterminer dans un diagramme pression-enthalpie du dioxyde de carbone qu'un mélange biphasique de dioxyde de carbone liquide et de dioxyde de carbone gazeux est formé immédiatement en aval de la buse avec une proportion massique de dioxyde de carbone liquide d'environ 63%. Après 3 heures de mise en oeuvre, la pompe de CO₂ est arrêtée et on diminue graduellement la pression dans l'enceinte jusqu'à pression atmosphérique en environ 30 minutes avant collecte du produit.

Un premier essai (Essai 1-1) est opéré en l'absence de lactose dans l'enceinte de détente.

Le second essai (Essai 1-2) est opéré avec 50,02 g de lactose (Tablettose 80) initialement placé dans l'enceinte de détente et maintenu sous agitation mécanique à 60 tours/min pendant toute la durée de l'essai.

### Résultats

L'essai 1-1 a conduit à la collecte de 1,82 g de poudre blanche fortement adhérente à la paroi du panier de collecte dans l'enceinte de détente.

L'essai 1-2 a conduit à la collecte de 51,67 g de formulation substance active - lactose sous forme d'une poudre s'écoulant facilement et présentant un aspect similaire à celui du lactose tel que reçu. La pesée du panier d'extraction après l'essai montre que 2,64 g de substance active a été extrait soit un rendement de collecte global de la formulation de 98% et un taux de charge massique théorique en substance active dans la formulation de 5,01%. Par dosage par chromatographie liquide à haute performance de 5 prélèvements de formulations, il est établi que le taux de charge massique réel en substance active dans la formulation est de 4,86%. Ce taux de charge massique expérimental correspond à un rendement de collecte en substance active au cours de l'opération de 97%. Par ailleurs, le coefficient de variation du taux de charge massique mesuré pour les 5 prélèvements est de 2,5%, ce qui indique une distribution très uniforme de la substance active dans la formulation.

L'analyse par chromatographie en phase liquide à haute performance indique une bonne stabilité de la substance active au cours du procédé pour les deux essais avec un une pureté et un profil de substances apparentées similaires à ceux du produit de départ pour les deux échantillons.

La caractérisation de l'état solide des particules de substance active de la formulation substance active-lactose par analyse enthalpique différentielle à balayage et diffraction RX montre que les particules de substance active sont cristallines et sous une forme cristalline similaires à celle du produit tel que reçu.

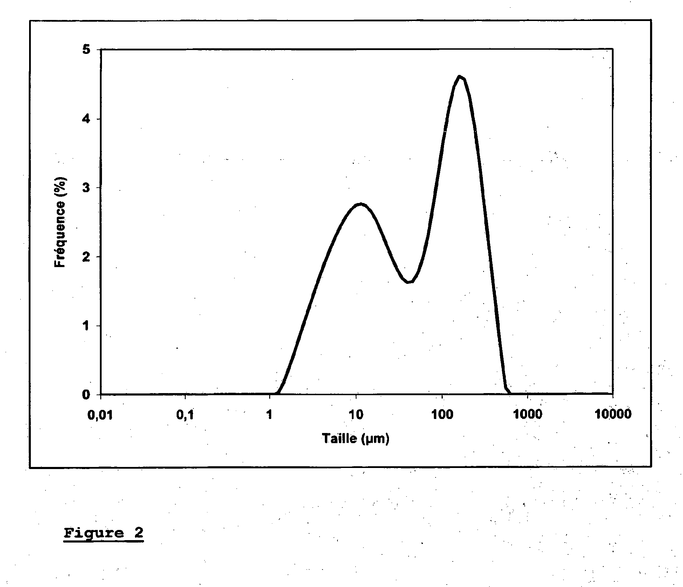

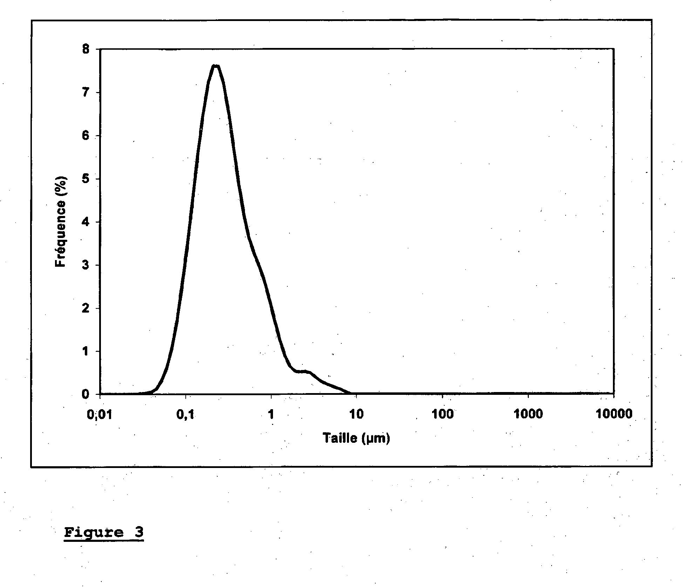

L'analyse de la taille des particules de substance active pour les deux essais est opérée par granulométrie laser en voie humide après dispersion des particules dans une solution d'eau saturée en substance active à température ambiante en présence de Tween 20 et après dissolution des éventuelles particules d'excipient. Là dispersion est opérée par passage au bain à ultrasons pendant 3 min avant mise en place de la dispersion dans l'appareil de mesure. Les profils de distribution granulométrique en volume obtenus pour les deux essais sont présentés respectivement sur la Figure 2 pour l'essai 1-1 et sur la Figure 3 pour l'essai 1-2.

Les résultats obtenus pour l'essai 1-1 mettent en évidence une distribution bimodale de la taille des particules avec le premier mode de la distribution centré à environ 10 µm et le second mode centré à environ 200 µm, soit des tailles de particules très éloignées du problème technique objet du procédé de l'invention. Par ailleurs, cette même analyse opérée sur 3 prélèvements distincts montre qu'en outre l'échantillon de substance active obtenu seul est peu homogène en distribution de tailles avec un diamètre médian (diamètre correspondant à la ligne des 50% sur la courbe de distribution cumulée en volume) moyen de 60,7 µm pour les 3 prélèvements avec un coefficient de variation de 19,9%.

Pour l'essai 1-2, il apparaît que les tailles de particules de substance active dans la formulation substance active - lactose suivent une distribution monomodale centrée entre 250 et 350 nm environ. L'analyse granulométrique menée sur 3 prélèvements distincts indique que la distribution de tailles des particules de substance active dans la formulation est très homogène avec un diamètre médian moyen de 282 nm avec un coefficient de variation de 1,5%.

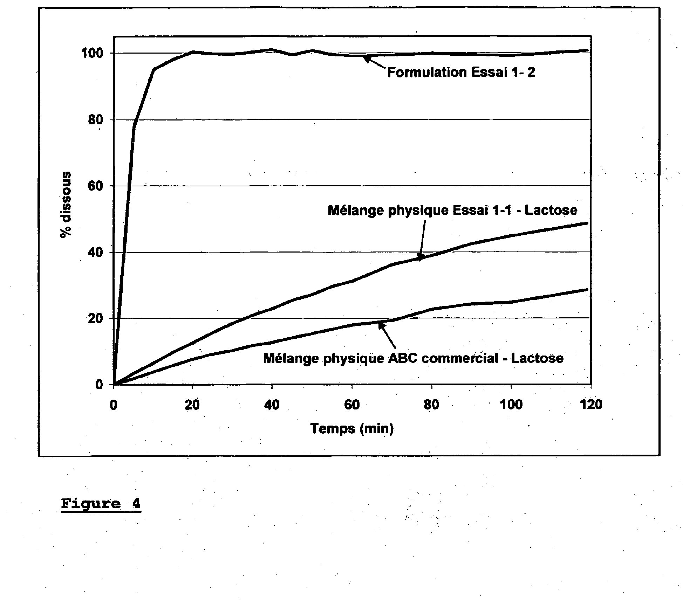

En vue de la réalisation de tests de dissolution *in vitro* opérés sur un appareil de type USP I, la formulation obtenue lors de l'essai 1-2 et deux mélanges physiques de substance active avec du lactose, l'un avec la substance active telle que reçue sous sa forme commerciale et l'autre avec la substance active obtenue lors de l'essai 1-1, sont conditionnés en gélules (LGA, taille 0, translucide, code 000020), en quantité équivalente à 5 mg, de substance active. Le milieu de dissolution était constitué d'une solution de SDS à 0,6% à une température de 37°C et la vitesse de rotation du panier était fixée à 80 tours/min. Les prélèvements ont été analysés par CLHP/UV après filtration. Les profils de dissolution *in vitro* présentés sur la Figure 4 montrent que la formulation obtenue lors de l'essai 1-2 a une cinétique de dissolution très largement améliorée par rapport aux mélanges physiques avec environ 95% de substance active dissous après 10 min contre moins de 10% pour les mélanges physiques.

Cet exemple montre que le procédé objet de l'invention permet l'obtention d'une formulation substance active - lactose au sein de laquelle la substance active est distribué de manière uniforme principalement sous forme de nanoparticules, avec en outre une cinétique de dissolution de la substance active en milieu aqueux très largement améliorée.

### Exemple 2 : Production d'une formulation Nifédipine-Mannitol

Le procédé de l'invention est mis en oeuvre sur un équipement correspondant au schéma décrit sur la Figure 1 pour la production de formulations Nifédipine - Mannitol avec un taux de charge massique en nifédipine visé de 5%.

1,5g de nifédipine mélangé à environ 20 g de billes de verre de 1 mm de diamètre est disposé dans un panier d'extraction de 20 mL. Ledit panier est placé à l'intérieur de l'autoclave d'extraction (5) chauffé à une température de 40°C. Après une étape de mise en régime, du dioxyde de carbone à l'état supercritique, porté à une pression de 25 MPa via la pompe (3) à un débit de 2 kg/h et à une température de 40°C via l'échangeur (4), percole à travers le panier d'extraction pour extraire la nifédipine. La solution de nifédipine dans le dioxyde de carbone supercritique ainsi formée est détendue à travers une buse de pulvérisation (8) constituée d'un capillaire en polyétheréthercétone (PEEK) de diamètre interne 150 µm et de longueur adaptée à la pression et au débit de travail. L'enceinte de détente (7), d'un volume intérieur utile de 310 mL, est chauffée à 40°C. La température de la solution supercritique immédiatement en amont de la buse est réglée à 105°C via le dispositif de chauffage (6). La pression au sein de l'enceinte de détente est réglée à 30 bar par une vanne automatique de régulation de la pression amont (11). Compte tenu des conditions de température et de pression immédiatement en amont de la buse de pulvérisation et de la pression réglée dans l'enceinte de détente, et sachant que le type de buse utilisé permet de considérer la détente comme quasi isenthalpe, il est possible de déterminer dans un diagramme pression-enthalpie du dioxyde de carbone qu'un mélange biphasique de dioxyde de carbone liquide et de dioxyde de carbone gazeux est formé immédiatement en aval de la buse avec une proportion massique de dioxyde de carbone liquide d'environ 6%.

Après 4 heures de mise en oeuvre, la pompe de CO₂ est arrêtée et on diminue graduellement la pression dans l'enceinte jusqu'à pression atmosphérique en environ 30 minutes avant collecte du produit.

Un premier essai (Essai 2-1) est opéré en l'absence de mannitol dans l'enceinte de détente.

Le second essai (Essai 2-2) est opéré avec 25,00 g de mannitol (Pearlitol 200 SD) initialement placé dans l'enceinte de détente et maintenu sous agitation mécanique à 120 tours/min pendant toute la durée de l'essai.

### Résultats

L'essai 2-1 a conduit à la collecte de 0,85g de poudre blanche principalement déposée sur le filtre placé au fond du panier de collecte dans l'enceinte de détente.

L'essai 2-2 a conduit à la collecte de 23,72 g de formulation nifédipine - mannitol sous forme d'une poudre s'écoulant facilement et présentant un aspect similaire à celui du mannitol seul. La pesée du panier d'extraction après l'essai montre que 1,07 g de nifédipine a été extrait soit un rendement de collecte global de la formulation de 91 % et un taux de charge massique théorique en nifédipine dans la formulation de 4,10 %. Par spectrophotométrie UV sur 5 prélèvements, il est établi que le taux de charge massique réel en nifédipine dans la formulation est de 3,89%. Ce taux de charge massique réel correspond à un rendement de collecte en nifédipine au cours du procédé de 95%. Par ailleurs, le coefficient de variation du taux de charge massique mesuré pour les 5 prélèvements est établi à 0,5%, ce qui indique une distribution très uniforme de la nifédipine dans la formulation.

L'analyse par chromatographie en phase liquide à haute performance indique une bonne stabilité de la nifédipine au cours du procédé pour les deux essais avec un titre et un profil de substances apparentées similaires à ceux du produit de départ pour les deux échantillons.

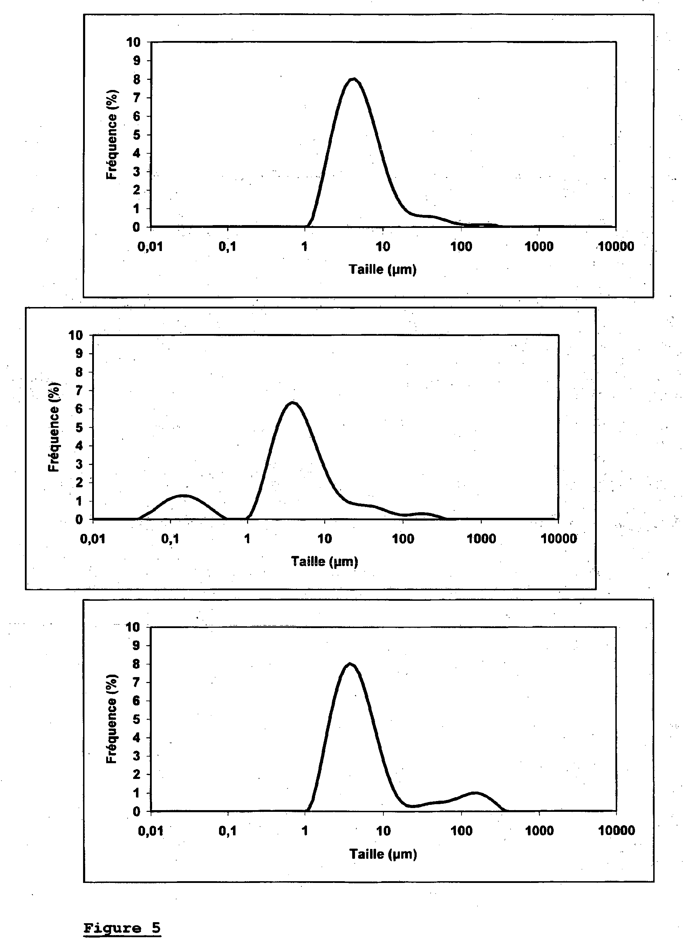

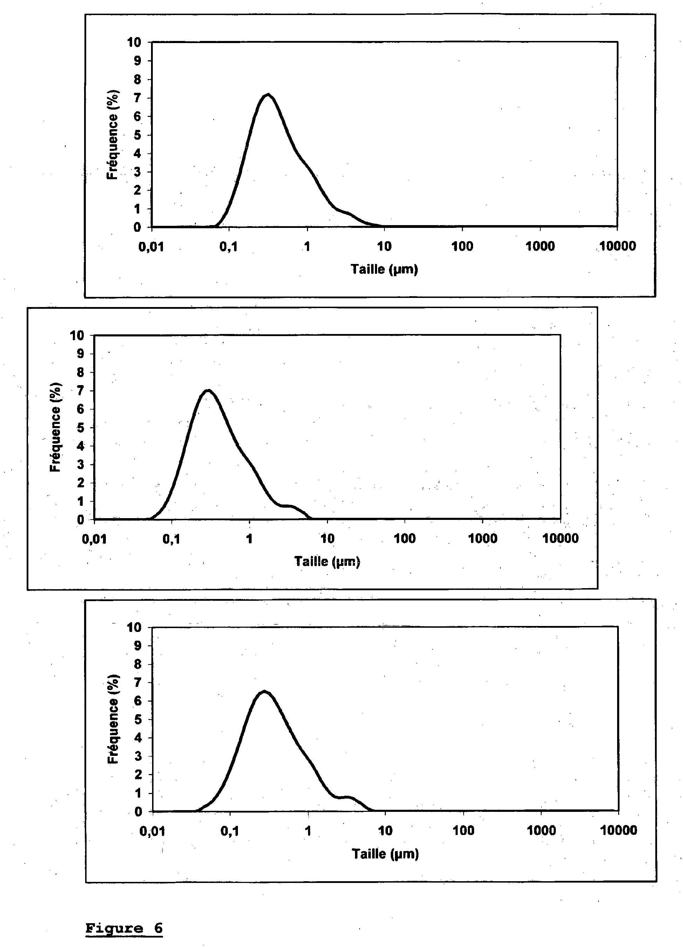

L'analyse de la taille des particules de nifédipine pour les deux essais est opérée par granulométrie laser en voie humide après dispersion des particules dans une solution d'eau saturée en nifédipine à température ambiante en présence de Tween 20 et après dissolution des éventuelles particules d'excipient. La dispersion des particules de nifédipine a été opérée manuellement par ajout d'une solution de Tween 20 dans l'eau dans un flacon dans lequel on a préalablement introduit le produit des essais suivi du retournement manuel du flacon de reconstitution, c'est-à-dire selon un protocole proche de ceux couramment utilisés pour la reconstitution extemporanée de formes injectables. Les profils de distribution granulométrique obtenus pour les deux essais sont présentés respectivement sur la Figure 5 pour l'essai 2-1 et sur la Figure 6 pour l'essai 2-2.

Les résultats obtenus pour l'essai 2-1 mettent en évidence une très grande hétérogénéité des distributions de tailles pour les 3 prélèvements opérés dans l'échantillon de nifédipine collecté en fin d'essai, avec une distribution soit monomodale, soit bimodale voire trimodale. Cependant pour les 3 prélèvements, il est possible de distinguer un pic principal dans la distribution des tailles centré à environ 4 µm.

Pour l'essai 2-2, les tailles de particules de nifédipine dans la formulation Nifédipine - Mannitol suit une distribution quasi-monomodale centrée à 300 nm environ. L'analyse granulométrique menée sur 3 prélèvements distincts indique que la distribution de tailles des particules de nifédipine dans la formulation est très homogène avec un diamètre médian moyen de 0,39 µm avec un coefficient de variation de 7,1 %. Il faut noter que l'homogénéité des distributions de tailles de particules de nifédipine dans la formulation et la valeur du coefficient de variation sont tout à fait acceptables compte tenu de la faible reproductibilité du mode de dispersion manuel mis en oeuvre. Des mesures granulométriques par spectroscopie à corrélation de photons confirment l'obtention de particules submicroniques de nifédipine pour l'essai 2-2.

Cet exemple montre donc que le procédé objet de l'invention permet l'obtention d'une formulation Nifédipine - Mannitol au sein de laquelle la nifédipine est distribuée de manière homogène et sous forme de particules de nifédipine principalement submicroniques.

### Exemple 3 : Production d'une formulation Sirolimus-Lactose

Le procédé de l'invention est mis en oeuvre sur un équipement correspondant au schéma décrit sur la Figure 1 pour la production de formulations Sirolimus - Lactose avec un taux de charge massique en sirolimus visé de 2%.

1,25 g de sirolimus mélangé à environ 20 g de billes de verre de 1 mm de diamètre est disposé dans un panier d'extraction de 20 mL. Ledit panier est placé à l'intérieur de l'autoclave d'extraction (5) chauffé à une température de 60°C. Après une étape de mise en régime, du dioxyde de carbone à l'état supercritique, porté à une pression de 33 MPa via la pompe (3) à un débit de 2 kg/h et à une température de 60°C via l'échangeur (4), percole à travers le panier d'extraction pour extraire le sirolimus. La solution de sirolimus dans le dioxyde de carbone supercritique ainsi formée est détendue à travers une buse de pulvérisation (8) constituée d'un capillaire en polyétheréthercétone (PEEK) de diamètre interne 127 µm et de longueur adaptée à la pression et au débit de travail. L'enceinte de détente (7), d'un volume intérieur utile de 310 mL, est chauffée à 60°C. La température de la solution supercritique immédiatement en amont de la buse est réglée à 60°C via le dispositif de chauffage (6). La pression au sein de l'enceinte de détente est réglée à 30 bar par une vanne automatique de régulation de la pression amont (11). Compte tenu des conditions de température et de pression immédiatement en amont de la buse de pulvérisation et de la pression réglée dans l'enceinte de détente, et sachant que le type de buse utilisé permet de considérer la détente comme quasi isenthalpe, il est possible de déterminer dans un diagramme pression-enthalpie du dioxyde de carbone qu'un mélange biphasique de dioxyde de carbone liquide et de dioxyde de carbone gazeux est formé immédiatement en aval de la buse avec une proportion massique de dioxyde de carbone liquide d'environ 51%.

Après 5 heures de mise en oeuvre, la pompe de CO2 est arrêtée et on diminue graduellement la pression dans l'enceinte jusqu'à pression atmosphérique en environ 30 minutes avant collecte du produit.

Un premier essai (Essai 3-1) est opéré en l'absence de lactose dans l'enceinte de détente.

Le second essai (Essai 3-2) est opéré avec 49,17 g de lactose (Pharmatose DCL 21) initialement placé dans l'enceinte de détente et maintenu sous agitation mécanique à 120 tours/min pendant toute la durée de l'essai.

### Résultats

L'essai 3-1 a conduit à la collecte de 0,555 g de poudre blanche en partie fortement adhérente à la paroi mais également sous la forme de particules assez grossières déposées sur le filtre dans le panier de collecte placé dans l'enceinte de détente.

L'essai 3-2 a conduit à la collecte de 49,77 g de formulation sirolimus - lactose sous forme d'une poudre s'écoulant facilement et présentant un aspect similaire à celui du lactose seul. Par dosage par chromatographie liquide à haute performance de 5 prélèvements, il est établi que le taux de charge massique réel en sirolimus dans la formulation est de 2,0% avec un coefficient de variation du taux de charge massique mesuré pour les 5 prélèvements de 2,3%, ce qui indique une distribution très uniforme du sirolimus dans la formulation.

L'analyse par chromatographie en phase liquide à haute performance indique une bonne stabilité du sirolimus au cours du procédé pour les deux essais avec un titre et un profil de substances apparentées similaires à ceux du produit de départ pour les deux échantillons.

La caractérisation de l'état solide des particules de sirolimus de la formulation sirolimus - lactose par analyse enthalpique différentielle à balayage montre que les particules de sirolimus sont cristallines et sous une forme cristalline similaire à celle du produit tel que reçu, soit la forme cristalline la plus stable.

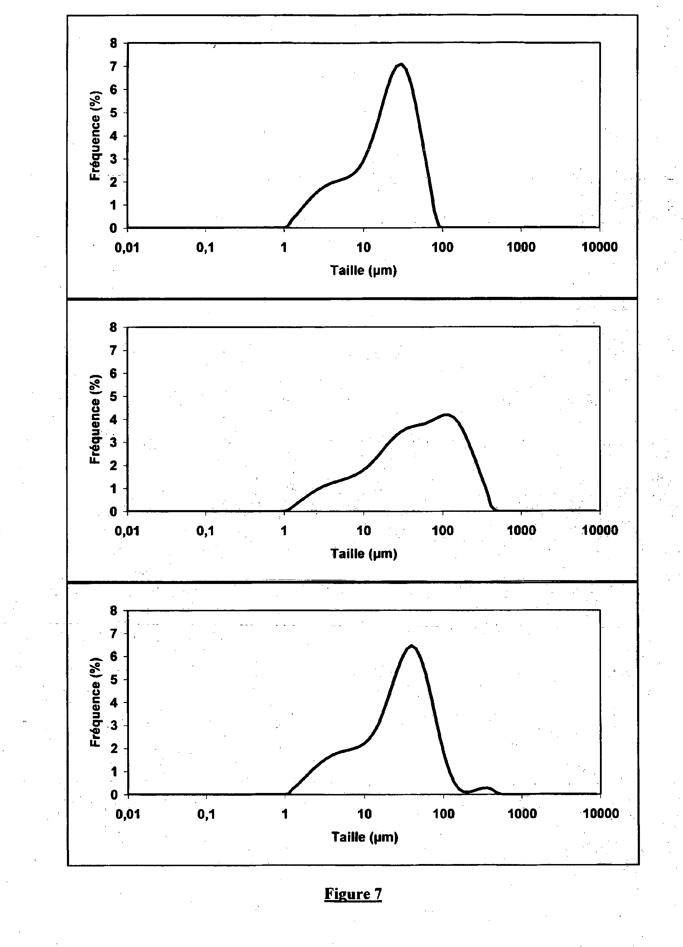

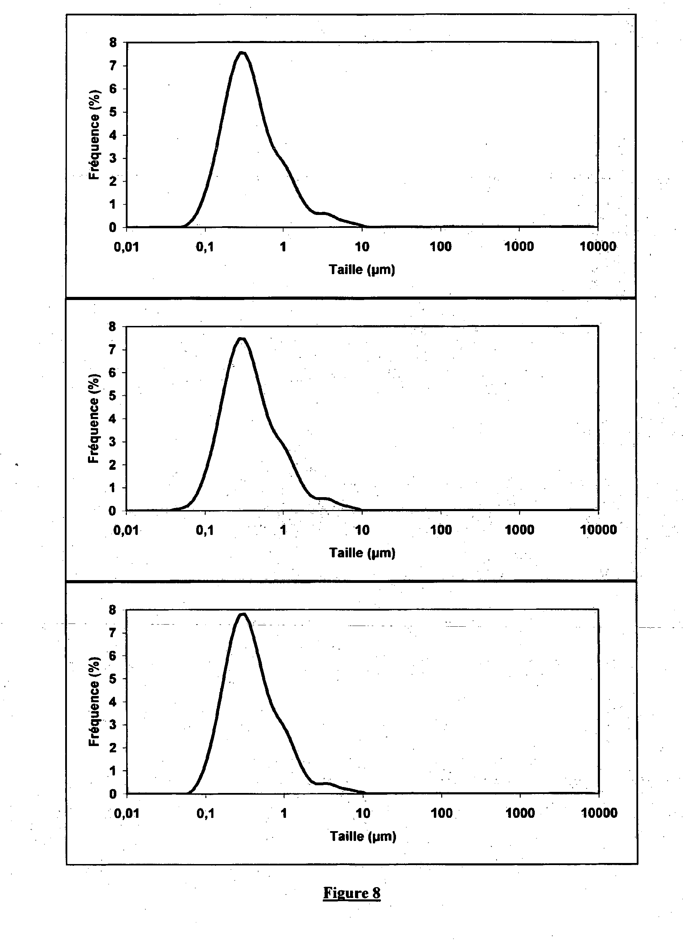

L'analyse de la taille des particules de sirolimus pour les deux essais est opérée par granulométrie laser en voie humide après dispersion des particules dans une solution d'eau saturée en sirolimus à température ambiante en présence de Tween 20 et après dissolution des éventuelles particules d'excipient. La dispersion est opérée par passage au bain à ultrasons pendant 3 min avant mise en place de la dispersion dans l'appareil de mesure. Les profils de distribution granulométrique obtenus pour les deux essais sont présentés respectivement sur la Figure 7 pour l'essai 3-1 et sur la Figure 8 pour l'essai 3-2.

Les résultats obtenus pour l'essai 3-1 mettent en évidence une très grande hétérogénéité des distributions de tailles pour les 3 prélèvements opérés dans l'échantillon de sirolimus collecté en fin d'essai. Pour les 3 prélèvements, il est néanmoins possible de déterminer un pic commun dans la distribution des tailles centré entre 30 et 40 µm. Il faut noter que pour l'un des prélèvements les tailles de particules mesurées sont particulièrement élevée avec environ 30 % des particules en volume d'un diamètre équivalent en volume supérieur à 100 µm. Cette mesure est cohérente avec l'observation visuelle de l'échantillon qui montre la présence de particules très grossières dans l'échantillon.

Pour l'essai 3-2, les tailles de particules de sirolimus dans la formulation Sirolimus - Lactose suit une distribution quasi-monomodale centrée à 300 nm environ. L'analyse granulométrique menée sur 3 prélèvements distincts indique que la distribution de tailles des particules de sirolimus dans la formulation est très homogène avec un diamètre médian moyen de 0,37 µm avec un coefficient de variation de 1,1 %. Des mesures granulométriques par spectroscopie à corrélation de photons confirment l'obtention de particules submicroniques de sirolimus pour l'essai 3-2.

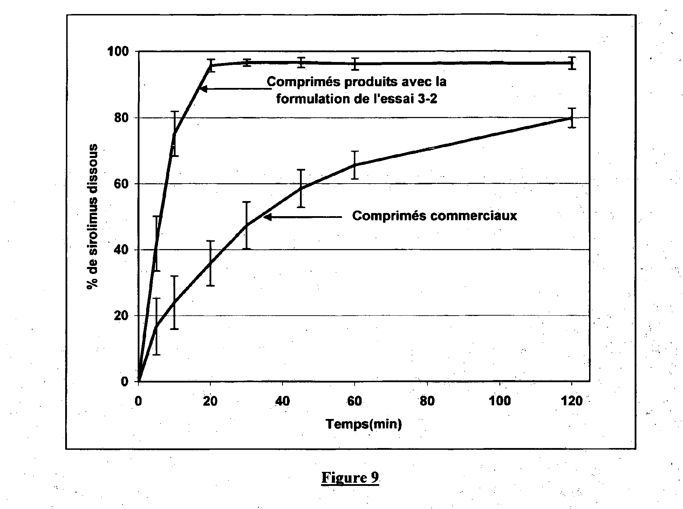

Des comprimés dosés à 2 mg en sirolimus sont fabriqués à partir de la formulation obtenue lors de l'essai 3-2, en vue de leur comparaison lors de tests de dissolution *in vitro* avec des comprimés commerciaux de Rapamune® 2 mg. Les tests de dissolution *in vitro* ont été opérés sur un appareil de type USP I avec un milieu de dissolution constitué d'une solution de Lauryl Sulfate de Sodium à 0,4%, à une température de 37°C et la vitesse de rotation du panier était fixée à 40 tours/min. Les prélèvements ont été analysés par CLHP/UV après filtration. Les profils de dissolution in vitro présentés sur la Figure 9 (valeurs moyennes pour 6 comprimés pour chaque courbe) montrent que les comprimés produits à partir de la formulation obtenue lors de l'essai 3-2 ont une cinétique de dissolution très largement améliorée par rapport aux comprimés commerciaux avec en moyenne environ 75% de sirolimus dessous après 10 min contre environ 25% pour les comprimés commerciaux.

Cet exemple montre donc que le procédé objet de l'invention permet l'obtention d'une formulation Sirolimus - Lactose au sein de laquelle le sirolimus est distribué de manière homogène et sous forme de particules de sirolimus principalement submicroniques. De plus, cette formulation Sirolimus - Lactose a permis de produire des comprimés présentant une cinétique de dissolution largement améliorée par rapport à des comprimés commerciaux.

## Revendications

1. Procédé de préparation d'une composition solide comprenant des particules fines, microniques et de préférence submicroniques d'au moins une substance active dispersées à la surface et/ou au sein d'un solide divisé **caractérisé en ce qu'**il comprend une étape consistant à détendre une solution de ladite substance active dans un fluide à pression supercritique, dans une enceinte à des conditions de pression et de température pour lesquelles au moins 1% du fluide, en masse par rapport à la masse de fluide total, se trouve sous forme liquide lors de la détente et **en ce que** ladite enceinte contient un solide divisé.

2. Le procédé selon la revendication **1 caractérisé en ce qu'**il comprend les étapes consistant à :
a) former une solution d'une substance active dans un fluide à pression supercritique ;
b) détendre ladite solution dans une enceinte dans des conditions de pression et de température pour lesquelles au moins 1% du fluide, en masse par rapport à la masse de fluide total, se trouve sous forme liquide lors de la détente ;
c) mettre en contact le fluide ainsi détendu avec un solide divisé dans ladite enceinte ;
d) récupérer un produit sec contenant ladite substance active sous forme de particules fines et ledit solide divisé.

3. Le procédé selon la revendication **2 caractérisé en ce qu'**il consiste à :
a) former une solution d'une substance active dans un fluide à pression supercritique ;
b) détendre ladite solution dans une enceinte dans des conditions de pression et de température pour lesquelles au moins 1% du fluide, en masse par rapport à la masse de fluide total se trouve sous forme liquide lors de la détente ;
c) mettre en contact le fluide ainsi détendu avec un solide divisé dans ladite enceinte ;
d) récupérer un produit sec contenant ladite substance active sous forme de particules fines et ledit solide divisé.

4. Le procédé selon l'une des revendications **1** à **3 caractérisé en ce que** le fluide à pression supercritique est choisi parmi le dioxyde de carbone, le protoxyde d'azote, les alcanes, les éthers, les hydrocarbures fluorés.

5. Le procédé selon l'une des revendications **1** à **4 caractérisé en ce que** ledit fluide à pression supercritique est du dioxyde de carbone à une pression comprise entre 7,4 MPa et 200 MPa, et à une température inférieure à 140°C.

6. Le procédé selon l'une des revendications **1** à **5 caractérisé en ce que** ledit fluide à pression supercritique est du dioxyde de carbone et **en ce que** la pression dans l'enceinte de détente est maintenue entre 0,52 et 7,4 MPa.

7. Le procédé selon l'une quelconque des revendications **1** à **6 caractérisé en ce que** la température du fluide à pression supercritique lors de l'étape de formation de la solution à pression supercritique est identique à la température immédiatement en amont du dispositif de détente.

8. Le procédé selon l'une quelconque des revendications **1** à **7 caractérisé en ce qu'**une partie du fluide qui se trouve à l'état liquide lors de la détente est vaporisée dans l'enceinte de détente.

9. Le procédé selon l'une quelconque des revendications **1** à **8 caractérisé en ce que** la totalité du fluide qui se trouve à l'état liquide lors de la détente est vaporisée dans l'enceinte de détente.

10. Le procédé selon l'une quelconque des revendications **1** à **9 caractérisé en ce que** le solide divisé est une poudre ou un matériau granulaire constitué d'au moins un excipient pharmaceutiquement acceptable.

11. Le procédé selon la revendication **10 caractérisé en ce que** ledit excipient est choisi parmi les excipients hydrophiles.

12. Le procédé selon la revendication **10 caractérisé en ce que** l'excipient est constitué d'une poudre composée d'excipient soluble dans les milieux aqueux.

13. Le procédé selon la revendication **10 caractérisé en ce que** l'excipient est choisi les sucres comme le lactose ou le sucrose, les polysaccharides comme la cellulose microcristalline, les dérivés de cellulose ou l'amidon, les polyols comme le mannitol, les lipides solides et les cires, les homopolymères et les copolymères solides comme les polyesters, les polyéthylène glycols, les poloxamères, les polyvinylpyrrolidones et dérivés, les composés inorganiques comme la silice.

14. Le procédé selon l'une quelconque des revendications **10** à **13 caractérisé en ce que** les particules d'excipient pharmaceutique ont une taille moyenne comprise entre 50 et 2 000 µm, de préférence entre 200 et 600 µm.

15. Le procédé selon l'une quelconque des revendications **1** à **14 caractérisé en ce que** la proportion massique de substances actives par rapport à l'excipient est compris entre 0,1 et 25%, de préférence entre 0,5 et 10%.

16. Le procédé selon l'une quelconque des revendications **1** à **15 caractérisé en ce que** l'on récupère une composition pharmaceutique solide contenant des particules de taille médiane inférieure à 1000 nm.

17. Le procédé selon l'une quelconque des revendications **1** à **16 caractérisé en ce que** l'on récupère une composition pharmaceutique solide contenant des particules essentiellement cristallines de substance active.

18. Le procédé selon la revendication **17 caractérisé en ce que** les particules cristallines de substance active sont essentiellement composées de la forme cristalline la plus stable.

19. Composition solide comprenant des particules fines, microniques et de préférence submicroniques d'au moins une substance active dispersées à la surface et/ou au sein d'un solide divisé **caractérisée en ce qu'**elle est obtenue par le procédé selon l'une quelconque des revendications **1** à **18.**

20. Composition selon la revendication **19,** dans laquelle le solide divisé est constitué de particules d'excipient pharmaceutique d'une taille moyenne comprise entre 50 et 2 000 µm, de préférence entre 200 et 600 µm.

21. Composition selon la revendication **19** ou la revendication **20,** dans laquelle le rapport massique entre la substance active et le solide divisé est compris entre 0,1 et 25%, de préférence entre 0.5 et 10%.

22. Composition selon l'une quelconque des revendications **19** à **21,** dans laquelle 90% des particules fines ont un diamètre volumique inférieur à 2 µm, de préférence, inférieur à 1000 nm.

23. Composition selon l'une quelconque des revendications **19** à **22,** dans laquelle les particules fines ont un diamètre volumique médian inférieur à 800 nm, de préférence, inférieur à 500 nm.

24. Composition selon l'une quelconque des revendications **19** à **23,** dans laquelle les particules fines sont des particules submicroniques cristallines, de préférence des particules cristallines contenant essentiellement la forme cristalline la plus stable.

## Patentansprüche

1. Verfahren zur Herstellung einer festen Zusammensetzung, umfassend feine, mikronische und vorzugsweise submikronische Partikel von mindestens einer aktiven Substanz, die auf der Oberfläche und/oder in einem getrennten Festkörper dispergiert sind, **dadurch gekennzeichnet, dass** es einen Schritt umfasst, der darin besteht, eine Lösung der aktiven Substanz in einem Fluid unter superkritischem Druck, in einem Behälter mit Druck- und Temperaturbedingungen auszudehnen, bei denen mindestens 1 Masse% des Fluids mit Bezug auf die gesamte Masse des Fluids in flüssiger Form bei der Ausdehnung vorliegt, und dadurch, dass der Behälter einen getrennten Feststoff enthält.

2. Verfahren nach Anspruch 1, **dadurch gekennzeichnet, dass** es die Schritte umfasst, die darin bestehen:
a) eine Lösung einer aktiven Substanz in einem Fluid unter superkritischem Druck zu bilden;
b) die Lösung in einem Behälter unter Druck- und Temperaturbedingungen auszudehnen, bei denen mindestens 1 Masse% des Fluids mit Bezug auf die gesamte Masse des Fluids bei der Ausdehnung in flüssiger Form vorliegt;
c) das so ausgedehnte Fluid mit einem getrennten Feststoff in dem Behälter in Kontakt zu bringen;
d) ein trockenes Produkt wiederzugewinnen, das die aktive Substanz in Form von feinen Partikeln und den getrennten Feststoff enthält.

3. Verfahren nach Anspruch **2, dadurch gekennzeichnet, dass** es darin besteht:
a) eine Lösung einer aktiven Substanz in einem Fluid unter superkritischem Druck zu bilden;
b) die Lösung in einem Behälter unter Druck- und Temperaturbedingungen auszudehnen, bei denen mindestens 1 Masse% des Fluids mit Bezug auf die Masse des gesamten Fluids bei der Ausdehnung in flüssiger Form vorliegt;
c) das so ausgedehnte Fluid mit einem getrennten Feststoff in dem Behälter in Kontakt zu bringen;
d) ein trockenes Produkt, das die aktive Substanz enthält in Form von feinen Partikeln und den getrennten Feststoff enthält, wiederzugewinnen.

4. Verfahren nach einem der Ansprüche **1** bis **3, dadurch gekennzeichnet, dass** das Fluid unter superkritischen Druck ausgewählt ist aus dem Kohlenstoffdioxid, dem Stickstoffprotoxid, den Alkanen, den Ethern, den fluorierten Kohlenwasserstoffen.

5. Verfahren nach einem der Ansprüche **1** bis **4, dadurch gekennzeichnet, dass** das Fluid unter superkritischen Druck Kohlenstoffdioxid unter einem Druck zwischen 7,4 MPa und 200 MPa und bei einer Temperatur von weniger als 140 °C ist.

6. Verfahren nach einem der Ansprüche **1** bis **5, dadurch gekennzeichnet, dass** das Fluid unter superkritischen Druck Kohlenstoffdioxid ist, und dadurch, dass der Druck im Ausdehnungsbehälter zwischen 0,52 und 7,4 MPa gehalten wird.

7. Verfahren nach einem der Ansprüche **1** bis **6, dadurch gekennzeichnet, dass** die Temperatur des Fluids unter superkritischem Druck beim Schritt des Bildens der Lösung unter superkritischem Druck identisch mit der Temperatur unmittelbar vorgelagert von der Ausdehnungsvorrichtung ist.

8. Verfahren nach einem der Ansprüche **1** bis **7, dadurch gekennzeichnet, dass** ein Teil des Fluids, der sich bei der Ausdehnung im flüssigen Zustand befindet, im Ausdehnungsbehälter verdampft wird.

9. Verfahren nach einem der Ansprüche **1** bis **8, dadurch gekennzeichnet, dass** die Gesamtheit des Fluids, die sich bei der Ausdehnung im flüssigen Zustand befindet, im Ausdehnungsbehälter zerstäubt wird.

10. Verfahren nach einem der Ansprüche **1** bis **9, dadurch gekennzeichnet, dass** der geteilte Feststoff ein Pulver oder ein granuliertes Material ist, das aus mindestens einem pharmazeutisch annehmbaren Trägerstoff besteht.

11. Verfahren nach Anspruch **10, dadurch gekennzeichnet, dass** der Trägerstoff ausgewählt ist aus den hydrophilen Trägerstoffen.

12. Verfahren nach Anspruch **10, dadurch gekennzeichnet, dass** der Trägerstoff aus einem zusammengesetzten Trägerstoffpulver besteht, das in wässrigen Medien löslich ist.

13. Verfahren nach Anspruch **10, dadurch gekennzeichnet, dass** der Trägerstoff ausgewählt ist aus den Zuckern wie z.B. Laktose oder Saccharose, den Polysacchariden wie z.B. der mikrokristallinen Zellulose, den Zellulosederivaten oder der Stärke, den Polyolen wie dem Mannitol, den festen Lipiden und den Wachsen, den Homopolymeren und den festen Copolymeren wie den Polyestern, den Polyethylenglykolen, den Poloxameren, den Polyvinylpyrrolidonen und Derivaten, den anorganischen Verbindungen wie dem Silizium.

14. Verfahren nach einem der Ansprüche **10** bis **13, dadurch gekennzeichnet, dass** die Partikel des pharmazeutischen Trägerstoffes eine mittlere Größe zwischen 50 und 2000 µm, vorzugsweise zwischen 200 und 600 µm aufweisen.

15. Verfahren nach einem der Ansprüche **1** bis **14, dadurch gekennzeichnet, dass** die Massenproportion von aktiven Substanzen mit Bezug auf den Trägerstoff zwischen 0,1 und 25 %, vorzugsweise zwischen 0,5 und 10 % liegt.

16. Verfahren nach einem der Ansprüche **1** bis **15, dadurch gekennzeichnet, dass** eine feste pharmazeutische Zusammensetzung wiedergewonnen wird, die Partikel mit einer mittleren Größe von weniger als 1000 nm enthält.

17. Verfahren nach einem der Ansprüche **1** bis **16, dadurch gekennzeichnet, dass** eine feste pharmazeutische Zusammensetzung wiedergewonnen wird, die im Wesentlichen kristalline Partikel der aktiven Substanz enthält.

18. Verfahren nach Anspruch **17, dadurch gekennzeichnet, dass** die kristallinen Partikel der aktiven Substanz im Wesentlichen aus der stabilsten kristallinen Form zusammengesetzt sind.

19. Feste Zusammensetzung, umfassend feine, mikronische und vorzugsweise submikronische Partikel von mindestens einer aktiven Substanz, die auf der Oberfläche und/oder in einem getrennten Festkörper dispergiert ist, **dadurch gekennzeichnet, dass** sie durch das Verfahren nach einem der Ansprüche **1** bis **18** erhalten wird.

20. Zusammensetzung nach Anspruch **19,** wobei der geteilte Feststoff aus Partikeln eines pharmazeutischen Trägerstoffs mit einer mittleren Größe zwischen 50 und 2000 µm, vorzugsweise zwischen 200 und 600 µm besteht.

21. Verfahren nach Anspruch **19** oder Anspruch **20,** wobei das Massenverhältnis zwischen der aktiven Substanz und dem getrennten Feststoff zwischen 0,1 und 25 % liegt, vorzugsweise zwischen 0,5 und 10%.

22. Zusammensetzung nach einem der Ansprüche **19** bis **21,** wobei 90 % der feinen Partikel einen Volumendurchmesser von weniger als 2 µm, vorzugsweise von weniger als 1000 nm aufweisen.

23. Zusammensetzung nach einem der Ansprüche **19** bis **22,** wobei die feinen Partikel einen mittleren Volumendurchmesser von weniger als 800 nm, vorzugsweise weniger als 500 nm aufweisen.

24. Zusammensetzung nach einem der Ansprüche **19** bis **23,** wobei die feinen Partikel kristalline submikronische Partikel sind, vorzugsweise kristalline Partikel, die im Wesentlichen die stabilste kristalline Form aufweisen.

## Claims

1. Process for preparing a solid composition comprising micronic and preferably submicronic fine particles of at least one active substance dispersed in the surface and/or within a divided solid, **characterized in that** it comprises a step consisting to expand a solution of said active substance in a fluid at supercritical pressure, in a chamber at conditions of pressure and temperature where at least 1% of the fluid, by mass relative to the total mass of fluid, is in liquid form during expansion and **in that** said chamber contains a divided solid.

2. The process according to claim **1 characterized in that** it comprises the steps consisting of:
a) forming a solution of an active substance in a fluid at supercritical pressure;
b) expanding said solution in a chamber in conditions of pressure and temperature where at least 1% of the fluid, by mass relative to the total mass of fluid, is in liquid form during expansion;
c) contacting the thus expanded fluid with a divided solid in said chamber;
d) recovering a dry product containing said active substance as fine particles and said divided solid.

3. The process according to claim **2 characterized in that** it consists of:
a) forming a solution of an active substance in a fluid at supercritical pressure;
b) expanding said solution in a chamber in conditions of pressure and temperature where at least 1% of the fluid, by mass relative to the total mass of fluid, is in liquid form during expansion;
c) contacting the thus expanded fluid with a divided solid in said chamber;
d) recovering a dry product containing said active substance as fine particles and said divided solid.

4. The process according to one of claims **1** to **3 characterized in that** the fluid at supercritical pressure is chosen from carbon dioxide, nitrous oxide, alkanes, ethers, fluorinated hydrocarbons.

5. The process according to one of claims **1** to **4 characterized in that** said fluid at supercritical pressure is carbon dioxide at a pressure between 7.4 MPa and 200 MPa, and at a temperature below 140 ° C.

6. The process according to one of claims **1** to **5 characterized in that** said fluid at supercritical pressure is carbon dioxide and **in that** the pressure in the expansion chamber is maintained between 0.52 and 7.4 MPa.

7. The process according to any one of claims **1** to **6 characterized in that** the temperature of the fluid at supercritical pressure during the solution forming at supercritical pressure step is identical to the temperature immediately upstream of the expansion device.

8. The process according to any one of claims **1** to **7 characterized in that** a portion of the fluid which is in the liquid state during expansion is vaporized into the expansion chamber.

9. The process according to any one of claims **1** to **8 characterized in that** all of the fluid which is in the liquid state during expansion is vaporized into the expansion chamber.

10. The process according to any one of claims **1** to **9 characterized in that** the divided solid is a powder or a granular material consisting of at least one pharmaceutically acceptable excipient.

11. The process according to claim **10 characterized in that** said excipient is chosen from hydrophilic excipients.

12. The process according to claim **10 characterized in that** the excipient consists of a powder composed of aqueous media soluble excipient.

13. The process according to claim **10 characterized in that** the excipient is selected sugars such as lactose or sucrose, polysaccharides such as microcrystalline cellulose, cellulose or starch derivatives, polyols such as mannitol, solid lipids and waxes, solid homopolymers and copolymers such as polyesters, polyethylene glycols, poloxamers, polyvinylpyrrolidones and their derivatives, inorganic compounds such as silica.

14. The process according to any one of claims **10** to **13 characterized in that** the pharmaceutical excipient particles have an average size between 50 and 2000 µm, preferably between 200 and 600 µm.

15. The process according to any one of claims **1** to **14 characterized in that** the mass proportion of active substances compared to excipient is between 0.1 and 25%, preferably between 0.5 and 10%.

16. The process according to any one of claims **1** to **15 characterized in that** a solid pharmaceutical composition is recovered, which contains particles having a median size less than 1000 nm.

17. The process according to any one of claims **1** to **16 characterized in that** that a solid pharmaceutical composition is recovered, which contains essentially crystalline particles of active substance.

18. The process according to claim **17 characterized in that** the crystalline particles of active substance are composed mainly of the most stable crystalline form.

19. A solid composition comprising micronic and preferably submicronic fine particles of at least one active substance dispersed in the surface and/or in a divided solid, **characterized in that** it is obtained by the process according to any one of claims **1** to **18.**

20. Composition of claim **19,** wherein the divided solid consists of pharmaceutical excipient particles having an average size between 50 and 2000 µm, preferably between 200 and 600 µm.

21. Composition of claim **19** or claim **20,** wherein the weight ratio between the active substance and the divided solid is between 0.1 and 25%, preferably between 0.5 and 10%.

22. Composition according to any one of claims **19** to **21,** wherein 90% of fine particles have a volume diameter of less than 2 µm, preferably less than 1000 nm.

23. Composition according to any one of claims **19** to **22,** wherein the fine particles have a median volume diameter of less than 800 nm, preferably less than 500 nm.

24. Composition according to any one of claims **19** to **23,** wherein the fine particles are crystalline submicronic particles, preferably crystalline particles containing essentially the most stable crystalline form.
